# EUROPEAN PATENT SPECIFICATION

(11) **EP 2 552 250 B1**
(45) Date of publication and mention of the grant of the patent: **26.02.2014**
(21) Application number: 11728391.1
(22) Date of filing: 28.03.2011
(51) Int. Cl.: A24B 15/28, A24B 15/30, A24D 1/02, A23L 1/226

(54) **SUPRAMOLECULAR COMPLEX FLAVOR IMMOBILIZATION AND CONTROLLED RELEASE**
AROMAIMMOBILISIERUNG EINES SUPRAMOLEKULAREN KOMPLEXES UND KONTROLLIERTE FREISETZUNG
IMMOBILISATION D'ARÔMES SOUS FORME DE COMPLEXES SUPRAMOLÉCULAIRES ET LIBÉRATION CONTRÔLÉE

(30) Priority: 26.03.2010 US 318226 P
(43) Date of publication of application: 06.02.2013
(73) Proprietor: Philip Morris Products S.A., 2000 Neuchâtel (CH)
(72) Inventor: MISHRA, Munmaya, K., Manakin Sabot, VA 23103 (US); DUAN, Biao, Appleton, WI 54913 (US); LIPOWICZ, Peter, J., Midlothian, VA 23112 (US); SWEENEY, William, R., Richmond, VA 23235 (US)
(74) Representative: Millburn, Julie Elizabeth
(86) International application number: PCT/IB2011/001152
(87) International publication number: WO 2011/117753

(56) References cited:
- EP-A2- 0 329 312
- EP-A2- 0 503 795

## Description

### SUMMARY

Flavoring substances can be added to tobacco products during manufacture. For example, menthol can be added to tobacco, which can be formed into tobacco rods. However, flavoring substances, such as menthol, can be volatile and can thus migrate from the region where applied, decreasing the flavor characteristics of the treated tobacco, and possibly deactivating sorbent materials which may be contained, for example, in cigarette filters. As a result, there is an interest in providing tobacco products in which flavoring substances are retained where deposited, and as a result, in which the flavor characteristics are predictable and consistent.

EP 0 329 312 A2 discloses a clathrate compound comprising a natural essential oil or a synthetic perfume and deoxycholic acid. EP 0 503 795 A2 discloses a smoking composition comprising an admixture of combustible filler and a flavorant-release additive which is a watersoluble molecular inclusion complex of a β-cyclodextrin derivative and a lipophilic flavorant compound.

The invention relates to a composition comprising a substrate material and a supramolecular complex, wherein the supramolecular complex comprises one or more flavorant compounds comprising at least one hydroxyl group or at least one aldehyde group, or both, non-covalently associated with a compound of Formula (I): 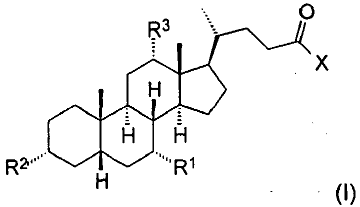 where R¹, R² and R³ are each independently selected from -H, -OH, and -OR, where R is selected from a straight or branched alkyl group of five carbons or less; X is OR⁴, where R⁴ is selected from hydrogen, or a straight or branched alkyl group of five carbons or less, or NR⁵R⁶, where R⁵ and R⁶ are each independently selected from hydrogen, straight or branched alkyl groups of five carbons or less.

The supramolecular complex can be disposed on or in the substrate material. In one embodiment, the substrate is a smokeable material, for example, a material comprising tobacco, a tobacco substitute, or a combination thereof. In an alternative embodiment, the substrate is a non-smokeable material, comprising a comestible product. In an alternative embodiment again, the substrate is a smokeless composition comprising tobacco, a tobacco substitute, or a combination thereof. When the substrate is used, the flavorant is released from the supramolecular complex.

Where the substrate is a smokeable material containing tobacco and/or a tobacco substitute, the composition may be provided as a component of a traditional cigarette, or an electrically heated cigarette. For example, the smokeable material may comprise a cigarette paper or wrapper on which the supramolecular complex has been printed.

The present invention also provides a filter for a smoking article, wherein the filter comprises the supramolecular complex.

The invention further provides a method for controlling release of one or more flavorant compounds comprising at least one hydroxyl group or at least one aldehyde group, or both, from a substrate by combining the substrate with an effective amount of a supramolecular complex described herein to form a treated substrate. The treated substrate is then heated or combusted to release the flavorant compound from the supramolecular complex. Alternatively, the treated substrate is contacted with saliva, releasing the flavorant compound into the saliva

A method for forming a flavorant supramolecular complex comprises combining a flavorant compound containing at least one hydroxyl or aldehyde group with a compound of Formula II: 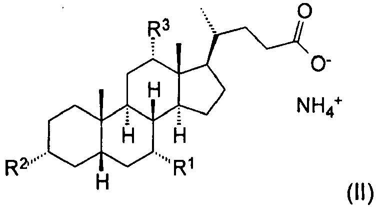 where R¹, R² and R³ are each independently selected from -H, -OH, and -OR, where R is selected from straight or branched alkyl groups of five carbons or less. It is desirable that this combining includes mixing and heating the flavorant compound and the compound of Formula II for a time and at a temperature effective to form a supramolecular complex as described herein. The mixing can occur in the presence or absence of a solvent. Preferably, the heating occurs under an inert atmosphere.

### DETAILED DESCRIPTION

The flavorant supramolecular complex and compositions containing it described herein are desirable because they provide releasable flavor or aroma characteristics to substrates that are released as the substrates are consumed or used. At the same time, these complexes minimize the transfer or loss of flavorant to other products, substrates, or packaging during storage. As a result, for example, a food or tobacco product containing a supramolecular complex of, e.g., menthol, would release menthol during use, when it is subjected to heat, hydrolysis, etc. However, the treated food or tobacco product would not transfer menthol to other food or tobacco products with which it is stored. Instead, the food or tobacco product would retain the menthol in the region where the supramolecular complex was applied. The resulting product provides greater flavor and/or aroma storage stability, and more predictable, consistent flavor and aroma characteristics.

The use of flavorant supramolecular complexes also provides for greater flexibility in combining or arranging the flavor or aroma characteristics of the treated substrate. This allows, for example, for the tailoring of flavor or aroma characteristics, so that, e.g., their perception by the user can vary during the use of the substrate. As a result; for example, a smokeless tobacco composition can be given a set of flavor characteristics that predominate when the product is first placed in the mouth, and that change over time as the product is chewed, or as supramolecular complexes having longer release profiles become hydrolyzed.

### Supramolecular Complexes

A supramolecular complex (SMC), also known as a clathrate, inclusion compound, or host-guest complex, is a multi-component system of atoms, ions, and/or molecules which are held together, at least in part, by non-covalent interactions such as hydrogen bonds, Van der Waals forces, pi-pi interactions, and/or electrostatic effects. These various attractive forces are far weaker than covalent bonding and thus supramolecular complexes are usually far less stable than compounds that are linked together entirely by covalent bonds. For example, SMC's are susceptible to being broken apart at elevated temperatures or when exposed to conditions which disrupt the weak bonding mechanisms that hold the complexes together. These can include acidic or alkaline conditions, hydrolysis, or solvation, e.g., by a polar solvent, which can disrupt the hydrogen bonding of the complexes.

When using the host-guest nomenclature to describe such a supramolecular complex, the larger compound is described as the "host" compound, and the smaller compound is described as the "guest" compound. In the present context, the flavorant compounds are all "guest" compounds, while the large, polycyclic compounds described by Formula I are all "host" compounds.

### Flavorants

In the present context, a flavorant compound is a molecular compound which imparts a desired flavor or aroma, or provides a desired chemesthetic effect. Any flavorant compound which is not damaged during SMC formation or flavorant release may be used, however preferred flavorant compounds contain one or more hydroxyl or aldehyde groups. Examples include, but are not limited to, vanillin, linalool, menthol, guaicol, thymol, coumarin, eugenol, cinnamaldehyde, and geraniol.

### Flavorant SMCs

The flavorant SMC's described herein comprise a compound of Formula I, given below, complexed with a flavorant compound as described above.

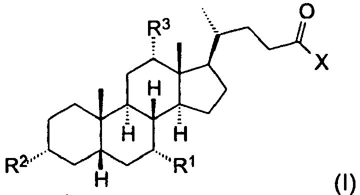

In this structure R¹, R² and R³ are each independently selected from -H, -OH, and -OR, where R is selected from straight or branched alkyl groups of five carbons or less; X is OR⁴, where R⁴ is selected from hydrogen, or a straight or branched alkyl group of five carbons or less, or is NR⁵R⁶, where R⁵ and R⁶ are each independently selected from hydrogen, straight or branched alkyl groups of five carbons or less.

### Forming Flavorant SMCs

Flavorant supramolecular complexes may be formed by a variety of methods. For example, a flavorant SMC may be formed by combining measured quantities of a flavorant compound, such as a flavorant containing one or more hydroxyl or aldehyde groups, and the host compound, such as a compound according to Formula (I) above or a derivative thereof, such as a carboxylic acid salt, and allowing these compounds to react, generally under an inert atmosphere and with appropriate mixing. This reaction can occur either neat (without solvent) or in a solvent.

The reaction may proceed at almost any temperature, although elevated temperatures are often used in order to speed the reaction or for other advantages. For example, if the host compound is solid at room temperature, running the reaction neat at room temperature could be impractical because the solid host compound does not mix or otherwise sufficiently interact with the flavorant compound. If the reaction is run at a temperature above the melting point of the higher melting material, typically of the host compound, the compounds mix and react readily. However, the flavorant SSC's often have a lower melting point than the host compound itself, so conducting the reaction at a temperature between the melting point of the flavorant SMC and the melting point of the host compound can provide a visual indication that reaction is proceeding, as well as provide a solvent in which the other components can dissolve, and thus react more readily.

One method for forming flavorant SMC's uses ammonium salts of carboxylic acid host compounds. In this method, the ammonium salt of a host compound of Formula II is combined with an appropriate flavorant compound.

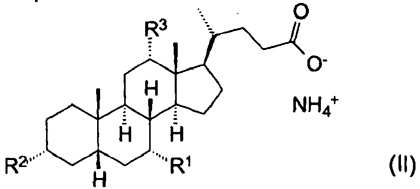

In this structure R¹, R² and R³ are each independently selected from -H, -OH, and -OR, where R is selected from straight or branched alkyl groups of five carbons or less. The resultant mixture is then heated and stirred (generally under an inert atmosphere). As the reaction proceeds, ammonia is liberated and the complex is formed.

Once flavorant SMC's have been formed, they can be stored, e.g., in dry containers at room temperature, for later use.

### Releasing Flavorant Compounds

In general, release of flavorant compounds from flavorant SMC's can result from any method which sufficiently disrupts the non-covalent interactions which hold together the flavorant compound and the host compound.

For example, at least some of these compounds are susceptible to flavorant release under pyrolytic conditions. The temperature required to cause such a release will generally be related to the strength of the non-covalent interactions. Thus, while a more weakly-interacting complex may release the flavorant compound at temperatures of 150° C or less, a different, more strongly-interacting, complex may require temperatures of 300° C or more to release the flavorant compound.

Additionally, particular flavorant SMCs may be sufficiently susceptible to flavorant release by hydrolysis that contact with water or water vapor is a practical method for releasing flavorant. Such contact can result in hydrolysis of the non-covalent linkage between the flavorant molecule and the host molecule. Hydrolysis can occur both at low to moderate temperatures, for example at room temperature, as well as at higher temperatures. Generally, release by hydrolysis will occur faster at higher temperatures. Release by hydrolysis can also be accomplished in the presence of water vapor. For example, one can initiate flavorant release by flowing moist air over or through flavorant SMCs, or compositions containing flavorant SMCs. This effect will be more pronounced at elevated temperatures.

Further, flavorant release can be accomplished by other methods which disrupt the non-covalent interactions that hold the flavorant molecule to the host molecule. These can include solvolysis, where the SMC is contacted with solvents that are adequately polar, adequately protic, or both, or by changing the pH of a solution of these SMCs. Photoirradiation may also cause release of the flavorant compound.

Flavorant release can also be enhanced or assisted by such means as mechanical action, such as mastication or suction. For example, if the flavorant SMC's are contained in a comestible, pharmaceutical formulation or other oral product, the flavorant can be released by chewing or sucking on the product.

In one embodiment, the compositions contain flavorant SMCs in amounts ranging between about 0.0001 and about 10 weight percent of the composition, based on the total weight of the composition. The resulting composition may combine the flavorant SMCs with a smokeable substrate, thereby forming a smoking composition, or may combine the flavorant SMC's with a substrate to form a non-smoking composition. Alternatively, the flavorant SMC's may be added as a particulate solid, for example in powder or granule form, to a substrate, such as a tobacco or non-tobacco substrate.

### Smoking Compositions

In one embodiment, a smoking composition is provided which comprises a mixture of a smokeable material and a flavorant SMC compound, where the flavorant SMC comprises a flavorant compound complexed with a host compound of Formula I.

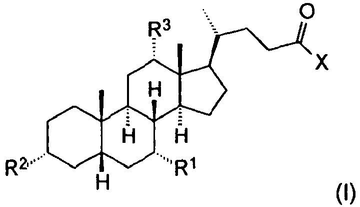

In this structure R¹, R² and R³ are each independently selected from -H, -OH, and -OR, where R is selected from straight or branched alkyl groups of five carbons or less; X is OR⁴, where R⁴ is selected from hydrogen, or a straight or branched alkyl group of five carbons or less, or NR⁵R⁶, where R⁵ and R⁶ are each independently selected from hydrogen, straight or branched alkyl groups of five carbons or less.

The flavorant SMC's may be combined with smokable substrate materials in a variety of ways. For example, the flavorant SMC's can be processed into solid shapes which can be placed at any desired location of a smoking article, such as a filter or tobacco rod for a cigarette, cigar, cigarillo or other similar smoking article. Alternatively or additionally, the flavorant SMC's can be dissolved in an appropriate solvent, such as ethanol or propylene glycol, or suspended in a liquid, and applied to the substrate, in particular to a smokable substrate, such as a tobacco or tobacco substitute, either before or after this substrate has been incorporated into a smoking article, for example, by spraying, dipping, or other method of solid-liquid contact. Suitable examples of tobacco substitute materials include sugar beet fiber, tea, herbs, vegetable material and combinations thereof.

Treated substrates, such as substrates treated by one of these methods, may also be used as a flavor enhanced materials which are later blended with other materials. For example, tobacco or tobacco substitute treated with a flavorant SMC as disclosed herein may be combined with other tobacco or tobacco substitute materials which have not been so treated, to form a blend thereof.

Release of the flavorant compound from the flavorant SMC's can be accomplished by pyrolysis in applications where the smoking composition is burned, as well as in applications where the smoking composition is only heated such as, for example, electrically heated cigarettes. Release from smoking compositions may also occur by hydrolysis, where heated water vapor in the air stream drawn through the smoking composition acts to release the flavorant compound from the host compound.

### Smoking Articles

In one embodiment, smoking articles are provided which comprise a smoking composition incorporating flavorant SMC's. As used herein, the term "smoking article" denotes any article that is generally consumed by burning or heating of a smoking composition, and includes articles wherein the smoking composition is heated but not combusted, and the heated vapors ingested, e.g. by inhaling them. It includes, but is not limited to, traditional cigarettes, cigars, cigarillos, pipes, electrically heated cigarettes, and the like. Preferred smoking articles are traditional cigarettes and electrically heated cigarettes.

As used herein, the term "traditional cigarette" denotes a cigarette that can be smoked by lighting an end of a wrapped rod of smoking composition and drawing air predominantly through the lit end by suction at a mouthpiece end of the cigarette. Traditional cigarettes can deliver smoke as a result of combustion of the smoking composition at temperatures that typically exceed about 800°C during a puff. Combustion can release constituents that are drawn through the cigarette, and can cool and condense to form aerosols. These aerosols can provide the flavors and aromas associated with smoking. Traditional cigarettes often contain a filter, composed of one or more pieces or plugs of filter material wrapped by a wrapper known as a plug wrap, which can typically be attached to one end of the rod of shredded smoking material (e.g., tobacco, also referred to as a "tobacco rod") by means of a wrapping or tipping material which may be made from paper, reconstituted tobacco sheets, natural leaf wrappers or other combustible sheet materials.. Either the rod of smoking material or the wrapper therefor or both can be burned or heated during use of the smoking article under smoking conditions.

As used herein, the term "electrically heated cigarette" denotes an alternative to the traditional cigarette used in used in electrical smoking systems. Electrical smoking systems can generally include an electrically powered lighter and an electrically heated cigarette, which can be constructed to cooperate with the lighter, and which generally contains a rod or column of smoking composition. Electrical smoking systems generate only small amounts of sidestream smoke, and also permit consumers to suspend and reinitiate smoking as desired. Exemplary electrical smoking systems are described in U.S. Patents Nos. 6,026,820; 5,988,176; 5,915,387; 5,692,526; 5,692,525; 5,666,976; 5,499,636; and 5,388,594. It is desirable that electrical smoking systems be capable of delivering smoke in a manner similar to the consumer's experiences with traditional cigarettes, such as by providing an immediacy response (smoke delivery occurring immediately upon draw), a desired level of delivery (that correlates with FTC tar level), a desired resistance to draw (RTD), as well as puff-to-puff and cigarette-to-cigarette consistency.

In a cigarette, cigar, cigarillo, EHC, or other similar smoking article, the flavorant SMC's may be incorporated in any of the portions of the smoking article. For example, the flavorant SMC's can be added to the bulk tobacco from which the tobacco rod of a cigarette is made. Additionally, the flavorant SMC's may be incorporated into the wrapper of a cigar, cigarette, cigarillo or other similar article. Different flavorant SMC's can be applied to the same smoking article, and can be applied in the same or different locations therein. For example, in one embodiment, different flavorant SMC's can be applied to different locations in the smoking article, so that as the article is consumed, release of different flavorant compounds occurs, and the user experiences different flavors and/or aromas at different times. This allows, for example, a flavorant SMC printed on cigarette paper near the filter section of the cigarette, so that consumption of the cigarette can conclude with a particular or unique flavor. The flavorant SMC can be applied to one or more portions of the smoking article with selectivity, and tends to be stable until encountering conditions that facilitate release of the flavorant during consumption. As a result, a smoking article can be produced in such a way that the consumer will not experience a particular flavor until using or consuming the particular portion of the substrate that has been treated with that particular flavorant SMC.

In another embodiment, combinations of different flavors can be obtained by blending different flavorant SMC's (e.g., SMC's containing different flavorant compounds) and placing these blends at various locations in the smoking article. Alternatively, SMC's having different release characteristics can be blended and disposed at locations within the smoking article, so that release of different flavorant compounds occurs at different times as the temperature or other release characteristics vary with time at a particular location.

In another embodiment, flavorant SMC's are applied to or incorporated in the wrapper of the smoking article. For example, the flavorant SMC's may be printed upon the wrapper of the smoking article. Further, the flavorant SMC's may be printed in distinct patterns on the wrapper to provide different effects when using the smoking article. For example, the flavorant SMC's can be printed on the wrapper near the filter end of the tobacco rod, which would provide a particular flavor when the smoking composition near the end of the smoking article is heated or burned. Additionally, multiple flavorant SMC's incorporating different flavorant compounds can be printed on the wrapper of the smoking article to provide different flavors at different times. This multiple flavor effect could also be produced by placing solid particles of different flavorant SMC's at different positions in the tobacco rod of the smoking article, as described in more detail below.

For example, the flavorant SMC's can be formed into a unitary solid and mechanically inserted into the tobacco rod of a cigar, cigarette, cigarillo, EHC, or other like article. Other manners of incorporating flavorant SMC's into a cigar, cigarette, cigarillo, EHC, or other like article are possible and are within the ambit of this disclosure.

More particularly, flavorant SMC's may be incorporated in the filter of a smoking article. The flavorant SMC can be added to any portion of the filter of a smoking article, including sorbent materials, such as carbon particles, present therein. For example, the carbon particles in the filter of a smoking article can be coated or partially coated with flavorant SMC's. Further, particles of flavorant SMC's can be placed in cavities within the filter. As mainstream smoke and aerosol containing water vapor passes though the filter of the smoking article, the flavorant compounds are released and provide flavor to the user.

Electrically heated cigarettes (EHC's), for example as described in U.S. Patent No. 5,692,525, the disclosure of which is hereby incorporated by reference in its entirety, are smoking articles that are used in combination with smoking systems which include electrically powered heaters that encompass a portion of the EHC. Once an EHC is inserted into an electrical smoking system, it is used in much the same fashion as a traditional cigarette, but without lighting or smoldering the cigarette. When an EHC is heated in its smoking system, it produces tobacco smoke, which the consumer then inhales.

EHC's may take a variety of forms, and may include some or all of the following parts: a cigarette paper overwrap; tobacco web; tobacco plug(s); filters of various forms; voids or air pockets. The cigarette paper overwrap, if used, provides the outermost surface for at least a portion of the length of the EHC. A tobacco web is a sheet material comprising tobacco particles and fibers, as well as other ingredients that function as binders, humectants or other functions. The tobacco web is often made into a cylindrical form surrounding, for at least part of the length of the EHC, all of the components of the EHC, except for the paper overwrap. Tobacco plugs are generally cylindrical agglomerations of tobacco. Tobacco plugs can be used in EHC's, and if used, will generally only extend through a portion of the length of the EHC. If used, the tobacco plug is the concentric center of the EHC cross section, and will be encircled by a tobacco web, a cigarette paper overwrap or both. Filters of a variety of forms, including free-flow filters, back flow filters and mouthpiece filters can be incorporated in an EHC. Voids or open spaces or air pockets can also be incorporated in the design of an EHC.

In an electrically heated cigarette, the flavorant SMC's may be incorporated in any of the portions of the EHC. For example, flavorant SMC's may be incorporated in the tobacco web, tobacco plug, paper overwrap, or in or on any of the filters used to make the EHC. Other manners of incorporating a flavorant SMC's into an electrically heated cigarette are possible and are within the ambit of this disclosure.

### Smokeless Tobacco Compositions

In one embodiment, flavorant SMC's can be made using components that are accepted food industry ingredients, and incorporated into a smokeless tobacco composition which comprises a substrate, such as tobacco or tobacco substitute, or a liner or pouch, which has been treated with one or more flavorant SMC's.

The flavorant SMC's may be incorporated into a smokeless tobacco composition in a variety of ways. For example, flavorant SMC's can be dissolved in an appropriate solvent or suspended in a liquid, and applied to the substrate, for example, by spraying. Further, the flavorant SMC's may be added as a powder to the substrate. Release of the flavor/fragrance can be accomplished by mastication, hydrolysis, or by some mixture of non-pyrolytic release mechanisms. Thus it would be possible using thee flavorant SMC's to produce, for example, a container enclosing multiple flavors of snuff, where each flavor is unaffected by the other flavors in the container, or which combine with the other flavors in a manner that is pleasing to the consumer. The amounts of flavorant SMC's included in the smokeless tobacco compositions can range from about 0.001 to about 10 % by weight of the total composition.

### Comestibles and Other Product for Oral Use

In another embodiment, flavorant SMC's can be made only using components that are generally recognized as safe (GRAS) and are used in comestibles. In one embodiment, the comestibles can contain about 0.0001 to about 10 % by weight of the flavorant SMC's, which may be added at any point in the process of making the foodstuffs to be treated, so long as the mixture is not subsequently subject to conditions, such as high temperatures, that might cause release of the flavorant compound(s) during manufacture. Release of the flavorant compounds can be accomplished by mastication, hydrolysis, or by some combination of non-pyrolytic release mechanisms. Thus, it would be possible to make, for example, a mint-flavored confection which does not flavor other mint-free confections with which it is packaged. In another embodiment, release of the flavorant compounds can be accomplished by heating, such as release of mint or other flavor in a hot chocolate, cocoa, coffee or tea drink.

In another embodiment, flavorant SMC's are used in other compositions for oral use, for example in lozenges, pharmaceutical formulations, dental floss, treatments and appliances, toothpicks, and other products meant to be used orally.

### Heatable or Combustible Items of Manufacture

The flavorant SMC described herein may be incorporated into heated or combustible products in order to release pleasant fragrances into the local atmosphere when the product is heated or burned. For example, by incorporating about 0.0001 to about 10% by weight of the polymeric flavor/fragrance compound into an otherwise unscented candle, a candle is produced which has little or no discernable fragrance before lighting, but releases a fragrance upon combustion. Other products contemplated incorporating a polymeric flavor/fragrance compound include fragrance sticks, incense, room deodorizers, artificial or treated fireplace logs and other products which are heated or combusted in a domestic or other environment for aesthetic reasons.

### Other Substrates

The flavorant SMC described herein may be used to treat other substrates where release of flavorants or aromas during use may be desirable. Examples include air or water filters, air purifying devices, and the like.

### Example 1 - Pyrolytic Release of Cinnamaldehyde from Cinnamaldehyde/Deoxvchoic Acid SMC

The release of cinnamaldehyde from cinnamaldehyde/deoxychoic acid supramolecular complex is observed by pyrolysis GC/MS and thermogravimetric analysis (TGA). No release is observed below a temperature of 350° C, whereupon cinnamaldehyde production is observed. Pyrolysis is performed at temperatures up to 500° C. With the exception of a small water peak at low GC retention times, no other detectable products are observed at any temperature.

### Example 2 - Release of Flavor Compounds from SMC's in Room Temperature Water

An SMC containing 30 wt% menthol and 70 wt% cholamide is prepared by combining cholamide with menthol and heating to around 70 °C. A pinch of this SMC containing menthol was added to room temperature tap water. Menthol flavor evolution was observed by smelling the water.

### Example 3 - Release of Flavor Compounds from SMC's in Hot Water

A pinch of the SMC containing menthol prepared in Example 2 is added to hot (~80° C) water. Spontaneous evolution of the menthol is observed by smell, which continued for hours.

### Example 4 - Pyrolytic Release of Menthol from Menthol/Cholamide SMC

The SMC containing menthol prepared in Example 2 is subjected to pyrolysis GC/MS and TGA. Menthol release was observed at temperatures as low as 150° C, and as high as 500° C.

It will be understood that the foregoing description is of the preferred embodiments, and it, therefore, merely representative of the articles and methods of manufacturing the same. It can be appreciated that variations and modifications of the different embodiments in light of the above teachings will be readily apparent to those skilled in the art.

## Claims

1. A composition comprising a smokeable material and a supramolecular complex, wherein the supramolecular complex comprises:
one or more flavorant compounds comprising at least one hydroxyl group or at least one aldehyde group, or both, non-covalently associated with
a compound of Formula (I): 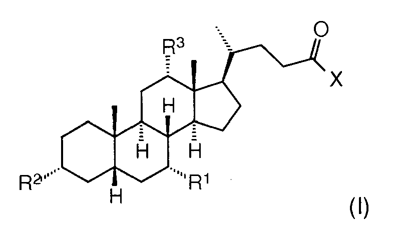 wherein R¹, R² and R³ are each independently selected from -H, -OH, or -OR, where R is selected from straight or branched alkyl groups of five carbons or less; and
wherein X is OR⁴, where R⁴ is selected from hydrogen, or a straight or branched alkyl group of five carbons or less, or is NR⁵R⁶, where R⁵ and R⁶ are each independently selected from hydrogen, straight or branched alkyl groups of five carbons or less.

2. A composition comprising a non-smokeable material and a supramolecular complex, wherein the non-smokeable material comprises a comestible product and the supramolecular complex comprises:
one or more flavorant compounds comprising at least one hydroxyl group or at least one aldehyde group, or both, non-covalently associated with
a compound of Formula (I): 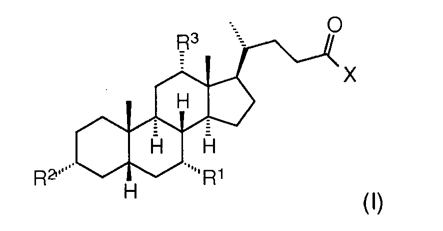 wherein R¹, R² and R³ are each independently selected from -H, -OH, or -OR, where R is selected from straight or branched alkyl groups of five carbons or less; and
wherein X is OR⁴, where R⁴ is selected from hydrogen, or a straight or branched alkyl group of five carbons or less, or is NR⁵R⁶, where R⁵ and R⁶ are each independently selected from hydrogen, straight or branched alkyl groups of five carbons or less.

3. A composition comprising a non-smokeable material and a supramolecular complex, wherein the non-smokeable material comprises a smokeless composition comprising tobacco, a tobacco substitute, or a combination thereof and the supramolecular complex comprises:
one or more flavorant compounds comprising at least one hydroxyl group or at least one aldehyde group, or both, non-covalently associated with
a compound of Formula (I): 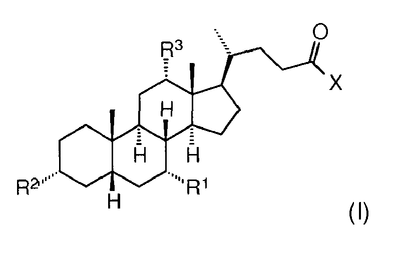 wherein R¹, R² and R³ are each independently selected from -H, -OH, or -OR, where R is selected from straight or branched alkyl groups of five carbons or less; and
wherein X is OR⁴, where R⁴ is selected from hydrogen, or a straight or branched alkyl group of five carbons or less, or is NR⁵R⁶, where R⁵ and R⁶ are each independently selected from hydrogen, straight or branched alkyl groups of five carbons or less.

4. The composition of claim 1, 2 or 3, wherein the flavorant compound is selected from the group consisting of vanillin, linalool, menthol, guaicol, thymol, coumarin, eugenol, cinnamaldehyde, and geraniol.

5. The composition of claim 1, 2 or 3, wherein the compound of Formula I is selected from the group consisting of cholic acid, deoxycholic acid, cholamide, and deoxycholamide.

6. The composition of claim 1, 2 or 3, wherein the supramolecular complex is in the form of a powder or granule.

7. The composition of claim 1, wherein the smokeable material comprises tobacco, a tobacco substitute, or a combination thereof as a component of a traditional cigarette.

8. The composition of claim 1, wherein the smokeable material is incorporated in a rod comprising tobacco, a tobacco substitute or a combination thereof as a component of an electrically heated cigarette.

9. The composition of claim 1, wherein the smokeable material comprises a cigarette paper on which has been printed the supramolecular complex.

10. A filter for a smoking article, the filter comprising a supramolecular complex, wherein the supramolecular complex comprises:
one or more flavorant compounds comprising at least one hydroxyl group or at least one aldehyde group, or both, non-covalently associated with
a compound of Formula (I): 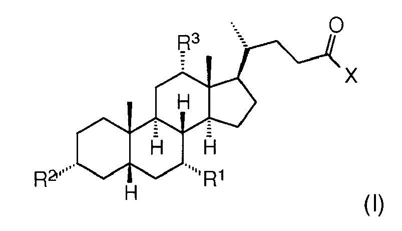 wherein R¹, R² and R³ are each independently selected from -H, -OH, or -OR, where R is selected from straight or branched alkyl groups of five carbons or less; and
wherein X is OR⁴, where R⁴ is selected from hydrogen, or a straight or branched alkyl group of five carbons or less, or is NR⁵R⁶, where R⁵ and R⁶ are each independently selected from hydrogen, straight or branched alkyl groups of five carbons or less.

11. A method for controlling release of one or more flavorant compounds comprising at least one hydroxyl group or at least one aldehyde group, or both, from a substrate, comprising:
combining the substrate with an effective amount of a supramolecular complex to form a treated substrate, wherein the supramolecular complex comprises:
one or more flavorant compounds comprising at least one hydroxyl group or at least one aldehyde group, or both, non-covalently associated with
a compound of Formula (I): 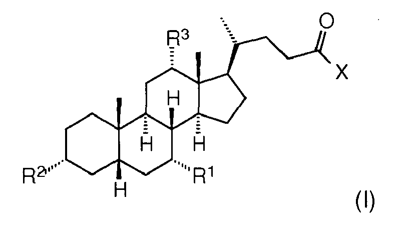
wherein R¹, R² and R³ are each independently selected from -H, -OH, or -OR, where R is selected from straight or branched alkyl groups of five carbons or less; and
wherein X is OR⁴, where R⁴ is selected from hydrogen, or a straight or branched alkyl group of five carbons or less, or is NR⁵R⁶, where R⁵ and R⁶ are each independently selected from hydrogen, straight or branched alkyl groups of five carbons or less;
heating or combusting the treated substrate or contacting the treated substrate with saliva; and
releasing the flavorant compound from the supramolecular complex.

## Patentansprüche

1. Zusammensetzung, die ein rauchbares Material und einen supramolekularen Komplex umfasst, wobei der supramolekulare Komplex Folgendes umfasst:
eine oder mehrere Aromaverbindungen, die wenigstens eine Hydroxylgruppe oder wenigstens eine Aldehydgruppe oder beides beinhalten, nicht-kovalent assoziiert mit
einer Verbindung der Formel (I): 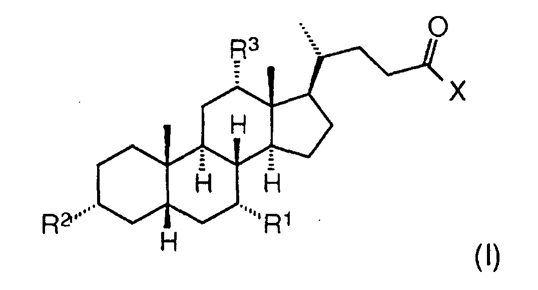 wobei R¹, R² und R³ jeweils unabhängig aus -H, -OH oder -OR ausgewählt sind, wobei R aus geraden oder verzweigten Alkylgruppen mit fünf Kohlenstoffatomen oder weniger ausgewählt ist; und
wobei X OR⁴ ist, wobei R⁴ aus Wasserstoff oder einer geraden oder verzweigten Alkylgruppe mit fünf Kohlenstoffatomen oder weniger ausgewählt ist, oder NR⁵R⁶ ist, wobei R⁵ und R⁶ jeweils unabhängig aus Wasserstoff, geraden oder verzweigten Alkylgruppen von fünf Kohlenstoffatomen oder weniger ausgewählt sind.

2. Zusammensetzung, die ein nicht rauchbares Material und einen supramolekularen Komplex umfasst, wobei das nicht rauchbare Material ein essbares Produkt umfasst und der supramolekulare Komplex Folgendes umfasst:
eine oder mehrere Aromaverbindungen, die wenigstens eine Hydroxylgruppe oder wenigstens eine Aldehydgruppe oder beide umfassen, nicht-kovalent assoziiert mit
einer Verbindung von Formel (I): 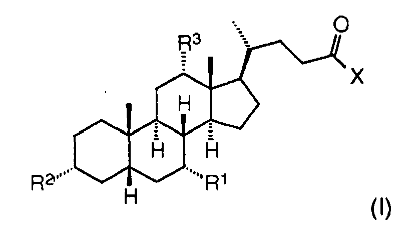 wobei R¹, R² und R³ jeweils unabhängig aus -H, -OH oder -OR ausgewählt sind, wobei R aus geraden oder verzweigten Alkylgruppen mit fünf Kohlenstoffatomen oder weniger ausgewählt ist; und
wobei X OR⁴ ist, wobei R⁴ aus Wasserstoff oder einer geraden oder verzweigten Alkylgruppe mit fünf Kohlenstoffatomen oder weniger ausgewählt ist, oder NR⁵R⁶ ist, wobei R⁵ und R⁶ jeweils unabhängig aus Wasserstoff, geraden oder verzweigten Alkylgruppen mit fünf Kohlenstoffatomen oder weniger ausgewählt sind.

3. Zusammensetzung, die ein nicht rauchbares Material und einen supramolekularen Komplex umfasst, wobei das nicht rauchbare Material eine rauchlose Zusammensetzung umfasst, die Tabak, einen Tabakersatz oder eine Kombination davon beinhaltet, und der supramolekulare Komplex Folgendes umfasst:
eine oder mehrere Aromaverbindungen, die wenigstens eine Hydroxylgruppe oder wenigstens eine Aldehydgruppe oder beides umfassen, nicht-kovalent assoziiert mit
einer Verbindung von Formel (I): 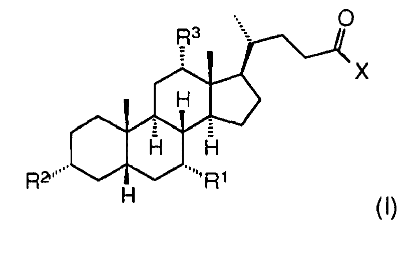 wobei R¹, R² und R³ jeweils unabhängig aus -H, -OH oder -OR ausgewählt sind, wobei R aus geraden oder verzweigten Alkylgruppen mit fünf Kohlenstoffatomen oder weniger ausgewählt ist; und
wobei X OR⁴ ist, wobei R⁴ aus Wasserstoff oder einer geraden oder verzweigten Alkylgruppe mit fünf Kohlenstoffatomen oder weniger ausgewählt ist, oder NR⁵ R⁶ ist, wobei R⁵ und R⁶ jeweils unabhängig aus Wasserstoff, geraden oder verzweigten Alkylgruppen mit fünf Kohlenstoffatomen oder weniger ausgewählt sind.

4. Zusammensetzung nach Anspruch 1, 2 oder 3, wobei die Aromaverbindung aus der Gruppe bestehend aus Vanillin, Linalool, Menthol, Guaicol, Thymol, Coumarin, Eugenol, Zimtaldehyd und Geraniol ausgewählt ist.

5. Zusammensetzung nach Anspruch 1, 2 oder 3, wobei die Verbindung von Formel I aus der Gruppe bestehend aus Cholsäure, Deoxycholsäure, Cholamid und Deoxycholamid ausgewählt ist.

6. Zusammensetzung nach Anspruch 1, 2 oder 3, wobei der supramolekulare Komplex in Form eines Pulvers oder Granulats vorliegt.

7. Zusammensetzung nach Anspruch 1, wobei der rauchbare Artikel Tabak, einen Tabakersatz oder eine Kombination davon als Komponente einer herkömmlichen Zigarette umfasst.

8. Zusammensetzung nach Anspruch 1, wobei der Rauchartikel in einen Stab integriert ist, der Tabak, einen Tabakersatz oder eine Kombination davon als Komponente einer elektrisch erhitzten Zigarette umfasst.

9. Zusammensetzung nach Anspruch 1, wobei der rauchbare Artikel ein Zigarettenpapier umfasst, auf dem der supramolekulare Komplex aufgedruckt ist.

10. Filter für einen Rauchartikel, wobei der Filter einen supramolekularen Komplex umfasst, wobei der supramolekulare Komplex Folgendes umfasst:
eine oder mehrere Aromaverbindungen, die wenigstens eine Hydroxylgruppe oder wenigstens eine Aldehydgruppe oder beide umfassen, nicht-kovalent assoziiert mit
einer Verbindung von Formel (I): 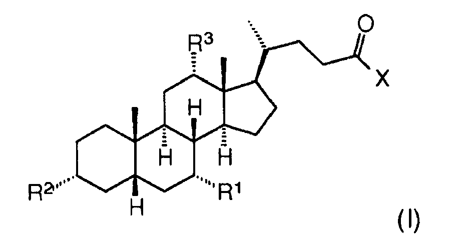 wobei R¹, R² und R³ jeweils unabhängig aus -H, -OH oder -OR ausgewählt sind, wobei R aus geraden oder verzweigten Alkylgruppen mit fünf Kohlenstoffatomen oder weniger ausgewählt ist; und
wobei X OR⁴ ist, wobei R⁴ aus Wasserstoff oder einer geraden oder verzweigten Alkylgruppe mit fünf Kohlenstoffatomen oder weniger ausgewählt ist, oder NR⁵R⁶ ist, wobei R⁵ und R⁶ jeweils unabhängig aus Wasserstoff, geraden oder verzweigten Alkylgruppen mit fünf Kohlenstoffatomen oder weniger ausgewählt sind.

11. Verfahren zum Regeln der Freisetzung von einer oder mehreren Aromaverbindungen, die wenigstens eine Hydroxylgruppe oder wenigstens eine Aldehydgruppe oder beides umfassen, von einem Substrat, das Folgendes beinhaltet:
Kombinieren des Substrats mit einer wirksamen Menge eines supramolekularen Komplexes zum Bilden eines behandelten Substrats, wobei der supramolekulare Komplex Folgendes umfasst:
eine oder mehrere Aromaverbindungen, die wenigstens eine Hydroxylgruppe oder wenigstens eine Aldehydgruppe oder beides umfassen, nicht-kovalent assoziiert mit
einer Verbindung von Formel (I): 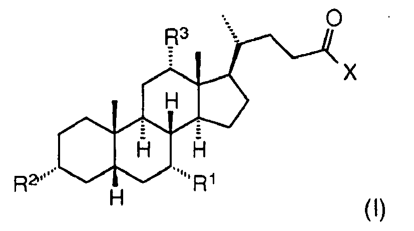
wobei R¹, R² und R³ jeweils unabhängig aus -H, -OH oder -OR ausgewählt sind, wobei R aus geraden oder verzweigten Alkylgruppen mit fünf Kohlenstoffatomen oder weniger ausgewählt ist; und
wobei X OR⁴ ist, wobei R⁴ aus Wasserstoff oder einer geraden oder verzweigten Alkylgruppe mit fünf Kohlenstoffatomen oder weniger ausgewählt ist, oder NR⁵R⁶ ist, wobei R⁵ und R⁶ jeweils unabhängig aus Wasserstoff, geraden oder verzweigten Alkylgruppen mit fünf Kohlenstoffatomen oder weniger ausgewählt sind;
Erhitzen oder Verbrennen des behandelten Substrats oder Inkontaktbringen des behandelten Substrats mit Speichel; und
Freisetzen der Aromaverbindung von dem supramolekularen Komplex.

## Revendications

1. Composition comprenant une substance fumable et un complexe supramoléculaire, le complexe supramoléculaire comprenant :
un ou plusieurs composants aromatiques comprenant au moins un groupe hydroxyle ou au moins un groupe aldéhyde, ou les deux, associés de manière non covalente avec
un composé de la Formule (I): 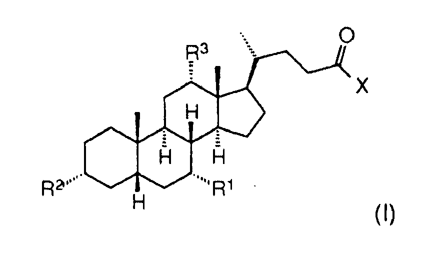 où R¹, R² et R³ sont chacun sélectionnés indépendamment parmi -H, -OH ou -OR, R étant sélectionné parmi des groupes alkyles linéaires ou ramifiés de cinq atomes de carbone ou moins ; et
où X est OR⁴, R⁴ étant sélectionné parmi l'hydrogène, ou un groupe alkyle linéaire ou ramifié de cinq atomes de carbone ou moins, ou est NR⁵R⁶, où R⁵ et R⁶ sont chacun sélectionnés indépendamment parmi l'hydrogène, ou des groupes alkyles linéaires ou ramifiés de cinq atomes de carbone ou moins.

2. Composition comprenant une substance non fumable et un complexe supramoléculaire, la substance non fumable comprenant un produit comestible et le complexe supramoléculaire comprenant :
un ou plusieurs composants aromatiques comprenant au moins un groupe hydroxyle ou au moins un groupe aldéhyde, ou les deux, associés de manière non covalente avec
un composé de la Formule (I) : 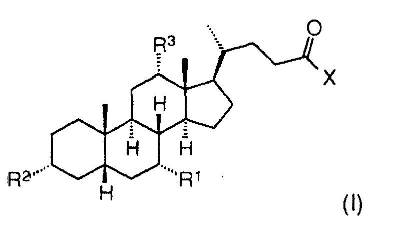 où R¹, R² et R³ sont chacun sélectionnés indépendamment parmi -H, -OH ou -OR, R étant sélectionné parmi des groupes alkyles linéaires ou ramifiés de cinq atomes de carbone ou moins ; et
où X est OR⁴, R⁴ étant sélectionné parmi l'hydrogène, ou un groupe alkyle linéaire ou ramifié de cinq atomes de carbone ou moins, ou est NR⁵R⁶, où R⁵ et R⁶ sont chacun sélectionnés indépendamment parmi l'hydrogène, ou des groupes alkyles linéaires ou ramifiés de cinq atomes de carbone ou moins.

3. Composition comprenant une substance non fumable et un complexe supramoléculaire, la substance non fumable comprenant une composition sans fumée comprenant du tabac, un substitut du tabac, ou une combinaison de ceux-ci, et le complexe supramoléculaire comprenant :
un ou plusieurs composants aromatiques comprenant au moins un groupe hydroxyle ou au moins un groupe aldéhyde, ou les deux, associés de manière non covalente avec
un composé de la Formule (I) : 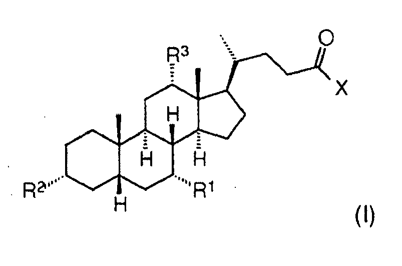 où R¹, R² et R³ sont chacun sélectionnés indépendamment parmi -H, -OH ou -OR, R étant sélectionné parmi des groupes alkyles linéaires ou ramifiés de cinq atomes de carbone ou moins ; et
où X est OR⁴, R⁴ étant sélectionné parmi l'hydrogène, ou un groupe alkyle linéaire ou ramifié de cinq atomes de carbone ou moins, ou est NR⁵R⁶, où R⁵ et R⁶ sont chacun sélectionnés indépendamment parmi l'hydrogène, ou des groupes alkyles linéaires ou ramifiés de cinq atomes de carbone ou moins.

4. Composition selon la revendication 1, 2 ou 3, dans laquelle le composé aromatique est sélectionné dans le groupe consistant en vanilline, linalool, menthol, guaiacol, thymol, coumarine, eugénol, cinnamaldéhyde et géraniol.

5. Composition selon la revendication 1, 2 ou 3, dans laquelle le composé de la Formule 1 est sélectionné dans le groupe consistant en acide cholique, acide désoxycholique, cholamide et désoxycholamide.

6. Composition selon la revendication 1, 2 ou 3, dans laquelle le complexe supramoléculaire se présente sous forme de poudre ou de granulé.

7. Composition selon la revendication 1, dans laquelle la matière fumable comprend du tabac, un substitut du tabac, ou une combinaison de ceux-ci comme composant d'une cigarette traditionnelle.

8. Composition selon la revendication 1, dans laquelle la substance fumable est incorporée dans un bâtonnet comprenant du tabac, un substitut du tabac, ou une combinaison de ceux-ci comme composant d'une cigarette chauffée électriquement.

9. Composition selon la revendication 1, dans lequel la substance fumable comprend un papier à cigarette sur lequel a été imprimé le complexe supramoléculaire.

10. Filtre d'article à fumer, le filtre comprenant un complexe supramoléculaire, dans lequel le complexe supramoléculaire comprend :
un ou plusieurs composants aromatiques comprenant au moins un groupe hydroxyle ou au moins un groupe aldéhyde, ou les deux, associés de manière non covalente avec
un composé de la Formule (I) : 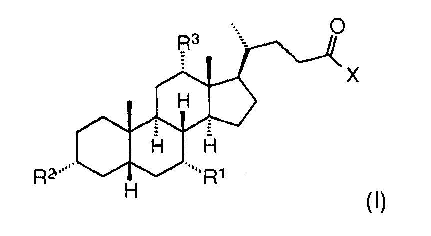 où R¹, R² et R³ sont chacun sélectionnés indépendamment parmi -H, -OH ou -OR, R étant sélectionné parmi des groupes alkyles linéaires ou ramifiés de cinq atomes de carbone ou moins ; et
où X est OR⁴, R⁴ étant sélectionné parmi l'hydrogène, ou un groupe alkyle linéaire ou ramifié de cinq atomes de carbone ou moins, ou est NR⁵R⁶, où R⁵ et R⁶ sont chacun sélectionnés indépendamment parmi l'hydrogène, ou des groupes alkyles linéaires ou ramifiés de cinq atomes de carbone ou moins.

11. Procédé de contrôle de la libération d'un ou plusieurs composés aromatiques comprenant au moins un groupe hydroxyle ou au moins un groupe aldéhyde, ou les deux, depuis un substrat, comprenant :
la combinaison du substrat à une quantité efficace d'un complexe supramoléculaire pour former un substrat traité, le complexe supramoléculaire comprenant :
un ou plusieurs composés aromatiques comprenant au moins un groupe hydroxyle ou au moins un groupe aldéhyde, ou les deux, associés de manière non covalente avec
un composé de la Formule (I) : 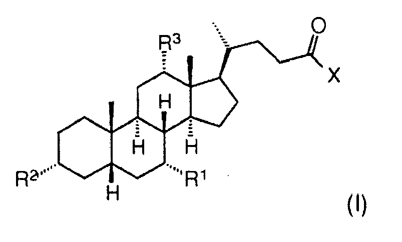
où R¹, R² et R³ sont chacun sélectionnés indépendamment parmi -H, -OH ou -OR, R étant sélectionné parmi des groupes alkyles linéaires ou ramifiés de cinq atomes de carbone ou moins ; et
où X est OR⁴, R⁴ étant sélectionné parmi l'hydrogène, ou un groupe alkyle linéaire ou ramifié de cinq atomes de carbone ou moins, ou est NR⁵R⁶, où R⁵ et R⁶ sont chacun sélectionnés indépendamment parmi l'hydrogène, ou des groupes alkyles linéaires ou ramifiés de cinq atomes de carbone ou moins ;
le chauffage ou la combustion du substrat traité ou la mise en contact du substrat traité avec la salive ; et
la libération du composé aromatique depuis le complexe supramoléculaire.
